# EUROPÄISCHE PATENTSCHRIFT

(11) **EP 2 328 472 B1**
(45) Veröffentlichungstag und Bekanntmachung des Hinweises auf die Patenterteilung: **08.03.2017**
(21) Anmeldenummer: 09748954.6
(22) Anmeldetag: 29.09.2009
(51) Int. Cl.: A61B 5/0476

(54) **VERFAHREN UND VORRICHTUNG ZUR AUSWERTUNG EINES NARKOSE- ODER INTENSIV-EEG**
METHOD AND DEVICE FOR EVALUATING AN INTENSIVE EEG OR AN EEG DURING ANAESTHESIA
PROCÉDÉ ET DISPOSITIF POUR ÉVALUER UN ÉLECTRO-ENCÉPHALOGRAMME PRATIQUÉ DANS LE CADRE D'UNE ANESTHÉSIE OU DE SOINS INTENSIFS

(30) Priorität: 29.09.2008 WO PCT/DE2008/001594
(43) Veröffentlichungstag der Anmeldung: 08.06.2011
(73) Patentinhaber: Schultz, Arthur, 29352 Adelheidsdorf (DE)
(72) Erfinder: Schultz, Arthur, 29352 Adelheidsdorf (DE)
(74) Vertreter: Günther, Constantin
(86) Internationale Anmeldenummer: PCT/DE2009/001367
(87) Internationale Veröffentlichungsnummer: WO 2010/034305

(56) Entgegenhaltungen:
- EP-A- 1 795 122
- EP-B- 0 856 181
- US-A- 4 678 865
- US-A- 4 846 190
- US-B1- 6 473 639
- US-B1- 6 731 975

## Beschreibung

Die Erfindung betrifft ein Verfahren zur Auswertung eines Narkose- oder Intensiv EEG gemäß Anspruch 1 und eine Vorrichtung zur Auswertung eines Narkose- oder Intensiv-EEG gemäß Anspruch 3.

Aus der EP 0 856 181 B1 sind ein Verfahren und eine Vorrichtung zur Auswertung eines Narkose- oder Intensiv-EEG bekannt. Aus EEG-Kurven wird mittels mathematisch-statistischer Methoden eine Klasseneinteilung des Narkose- oder Intensiv-EEG vorgenommen. Darüber hinaus werden Artefakte erfasst und zur Korrektur oder Unterdrückung von Klasseneinteilungen berücksichtigt.

Aus der EP 1 795 122 A1 ist es bekannt, EEG-Signale und Bioimpedanz-Signale zu registrieren. Überschreitungen eines Schwellwertes der Bioimpedanz oder die Ähnlichkeit der Signale im EEG- und im Bioimpedanz-Signal werden zur Artefakterkennung genutzt. Mit Hilfe der Bioimpedanzanalyse können Artefakt belastete EEG-Signalabschnitte verworfen werden oder die Klassifikation als "wach" kann unterstützt werden, zum Beispiel bei Erkennung von Augenzwinkern. Vornehmlich geht es bei dieser Druckschrift um Artefakte im Gesichtsbereich.

Aus der US 6,731,975 A ist ein Verfahren bekannt, den zerebralen Status eines Patienten zum Beispiel nach Gabe eines

Anästhetikums zu bestimmen. Dazu werden die Entropie des EEG-Signals und des kombinierten EEG-EMG-Signals sowie ein reiner EMG-Index berechnet.

Aus der US 4,846,190 ist ein enzephalographisches System bekannt, bei dem Hirnwellen eines Patienten erfasst und bearbeitet werden, um elektrische Interferenzen, Muskelartefakte oder andere Störungen zu verringern.

Es hat sich gezeigt, dass es eine Reihe von Auslösern für Biosignale gibt, die Veränderungen in EEG-Kurven hervorrufen und eine Klasseneinteilung des Narkose- oder Intensiv-EEG stören können. Hervorzuheben sind dabei volatile Anästhetika auf Fluranbasis, insbesondere Sevofluran, die mit zunehmender Dosierung Krampfpotentiale auslösen können.

Der Erfindung liegt die Aufgabe zugrunde, bei der Auswertung eines Narkose- oder Intensiv-EEG Biosignale erkennen und von Artefakten unterscheiden zu können.

Diese Aufgabe wird mit einem Verfahren zur Auswertung eines Narkose- oder Intensiv-EEG gemäß Anspruch 1 und einer Vorrichtung zur Auswertung eines Narkoseoder Intensiv-EEG gemäß Anspruch 3 gelöst.

Weiterbildungen und vorteilhafte Ausgestaltungen ergeben sich den jeweiligen Unteransprüchen.

Bei der erfindungsgemäßen Lösung werden weiterhin aus den EEG-Kurvenverläufen Parameter aus dem Zeit- und/oder Frequenzbereich ermittelt und in multivariate Klassifikationsfunktionen eingesetzt. Dabei wird automatisch eine Einteilung in Stadien eines Narkose- oder Intensiv-EEG vorgenommen. Darüber hinaus werden die Kurvenverläufe aber auch auf einstreuende Signalanteile untersucht. Einstreuende Signalanteile sind solche, die den Gesamtkurvenverlauf beeinflussen, aber untypisch für ein Narkose- oder Intensiv-EEG sind und deren Ursprung auch ein anderer ist als der für die Narkose- oder Intensiv-EEG typischen oder charakteristischen Kurven.

Bei den Einstreuungen handelt es sich um Biosignale mit Epilepsie-typischen Kurvenverläufen in Form von Krampfpotentialen oder um Artefakte die jeweils Änderungen der Kurvenverläufe des eigentlichen Narkose- oder Intensiv-EEG hervorrufen. Um herauszufinden, ob es sich bei den Einstreuungen um Biosignale und/oder Artefakte handeln könnte, wird bei Nachweis einstreuender Signale zusätzlich eine Artefaktanalyse durchgeführt. Für eine Artefaktanalyse können an sich bekannte Verfahren und Methoden genutzt werden. Ergibt die Auswertung, dass keine Artefakte vorliegen, wird im Umkehrschluss gefolgert, dass die Einstreuungen von Biosignalen nicht Narkose- oder Intensiv-EEG charakteristischer Kurven stammen. Werden hingegen Artefakte nachgewiesen, so können die Einstreuungen ausschließlich durch Artefakte oder durch die Kombination von Artefakten und Biosignalen hervorgerufen werden. Ein eindeutiger Nachweis der Existenz von Biosignalen ist in diesem Fall nicht möglich und es werden somit beide Fälle gleich behandelt, indem die Existenz von Biosignalen bei Nachweis von Artefakten nicht verifiziert wird.

Bei Verifizierung von Biosignalen kann die Analyse dieser Biosignale nicht Narkose- oder Intensiv-EEG charakteristischer Kurven durch Vergleich der durch die einstreuenden Biosignale generierten EEG-Kurven mit übereinstimmenden Merkmalen gespeicherter Grafikmuster von Kurvenverlaufen unterschiedlichen Bioursprungs durchgeführt werden.

Es hat sich herausgestellt, dass nicht alle Biosignale sich gleichermaßen charakterisieren lassen, indem Parameter aus dem Zeit- und/oder Frequenzbereich ermittelt und in multivariate Funktionen eingesetzt werden.

Dies gilt insbesondere für Biosignale, deren Verlauf von dem Kurvenverlauf eines Narkose- oder Intensiv-EEG abweicht. In diesem Fall ist eine andere Art der Analyse, nämlich ein grafischer Vergleich des Kurvenverlaufs einstreuender Biosignale mit gespeicherten Mustern von Signalverläufen unterschiedlicher Biosignale aussagekräftiger. Durch Vergleich von Merkmalen gespeicherter Grafikmuster können die einstreuenden Biosignale dann einem gespeicherten Muster zugeordnet werden, wobei über die Verknüpfung des bekannten Ursprungs des gespeicherten Musters auch auf den Ursprung des analysierten Biosignais geschlossen werden kann. Erfingdungsgemäß werden bei Existenz von Biosignalen diese Signalanteile auf Epilepsie-typische Kurvenverläufe in Form von Krampfpotentialen analysiert. Dadurch ist es möglich, bei einem Narkose- oder Intensiv-EEG, das Einstreuungen von weiteren Biosignalen zeigt, mögliche Epilepsie-typische Einstreuungen zu erkennen, die durch verabreichte Medikamente verursacht werden können.

Als Artefakte werden Formänderungen von Ableitungselektroden erfasst und ausgewertet.

Solche Formänderungen können die von den Ableitungselektroden abgeleiteten Spannungen aufgrund von Ladungsverschiebungen und von Änderungen der Leitfähigkeit zwischen Ableitungselektrode und Haut beeinflussen. Durch Erfassung von Formänderungen können zeitgleiche Beeinflussungen der abgeleiteten Spannungen als Artefakte erkannt werden.

Biosignale, die nicht Narkose- oder Intensiv-EEG charakteristische Kurven aufweisen, zum Beispiel Epilepsie-typische Kurvenformen, können sich durch steile Amplituden-Zeitübergänge auszeichnen, wie sie auch durch Artefakte an den Ableitungselektroden auftreten können, zum Beispiel wenn die Elektroden oder deren Leitungen mechanisch bewegt werden.

Durch Erfassung von Formänderungen von Ableitungselektroden lassen sich die in der Praxis häufigsten Artefakte erkennen und als Nachweis auswerten, ob einstreuende Signale Artefakte oder Biosignale sind.

Durch einen direkten qualitativen oder auch quantitativen Vergleich wird die Verifizierung sicherer.

Die Verformungssensoren zur Erfassung von Formänderungssignalen können Kapazitäten umfassen, die durch Verformung veränderbar sind und deren Kapazitätsänderung mit der Verformung der EEG-Elektroden korreliert.

Durch Kapazitäten als Verformungssensoren ist eine Erfassung und Verstärkung von Formänderungssignalen mit dem üblichen EEG-Signalverstärker und anschließender rechner-gestützten Auswertung möglich.

Die Kapazitäten bestehen vorzugsweise aus benachbarten Anschlussleitungen und zwischen den Anschlussleitungen angeordneten, jeweils ein Dielektrikum darstellenden Isolierungen.

Auf diese Weise können Leiter-Dielektrikum-Komponenten einer Mehrelektrodenanordnung zusätzlich als Verformungssensoren genutzt werden.

Ergänzend können die EEG-Elektroden Hautpräparationsmittel umfassen, die verformungsabhängige Leitwertänderungen zwischen der Elektrodenoberfläche und der Haut mindern.

Durch die Hautpräparationsmittel wird ein Einfluss von Änderungen der Leitfähigkeit zwischen Ableitungselektrode und Haut auf die von den Ableitungselektroden abgeleiteten Spannungen reduziert.

Die Hautpräparationsmittel sind vorzugsweise Oberflächen vergrößernde Ein- und/oder Ausstülpungen der Elektrodenoberfläche.

Durch die Hautpräparationsmittel wird die Hautoberfläche an der Kontaktstelle der Elektrode durch Abtrag und Aufbruch für tieferes Eindringen von leitfähigem Elektrodengel zugänglich. Dadurch wird die effektive Kontaktfläche erhöht, wodurch verformungsabhängige Leitwertänderungen von Teilen der Kontaktfläche weniger Einfluss auf die gesamte Leitfähigkeit haben.

Nachfolgend wird die Erfindung anhand eines Flussdiagramms und eines Ausführungsbeispiels erläutert, das in der Zeichnung dargestellt ist.

In der Zeichnung zeigen
Fig. 1 ein Flussdiagramm der Erfindung und
Fig. 2 eine Vorrichtung aus einem Rechner und einer Messeinheit für einen EEG-Kanal.

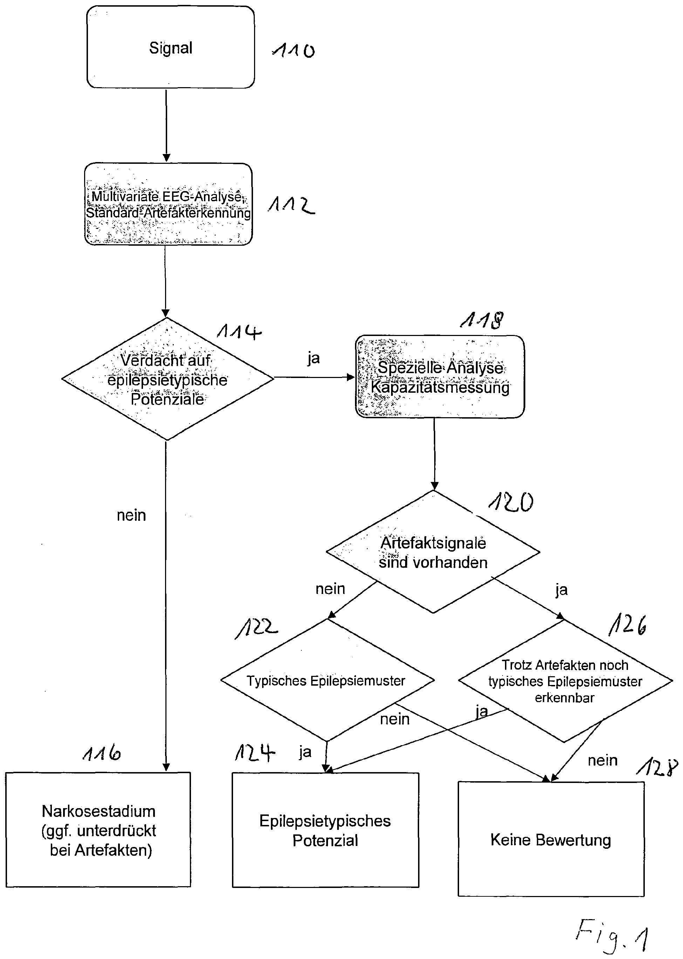

Gemäß dem in Fig. 1 dargestellten Flussdiagramm wird ein eintreffendes Signal 110 in Analyseschritten 112 dadurch analysiert, dass Parameter im Zeit- und/oder Frequenzbereich in multivariate Klassifikationsfunktionen eingesetzt werden und darauf automatisch eine Stadieneinteilung eines Narkose- oder Intensiv-EEG vorgenommen wird. Zusätzlich findet hier auch eine Standard-Artefakterkennung statt, unter Berücksichtigung derer eine Klasseneinteilung unterdrückt oder korrigiert werden kann.

In einem nachfolgenden Schritt 114 wird das Signal auf mögliche Einstreuungen analysiert, wobei die Einstreuungen Signalanteile sind, die für ein Narkose- oder Intensiv-EEG nicht charakteristisch sind und daher eine Klasseneinteilung stören oder verfälschen würden. Im vorliegenden Beispiel geht es um mögliche Epilepsie-typische Potentiale.

Werden keine Einstreuungen festgestellt, wird bei 116 das Stadium des Narkose- und Intensiv-EEG dargestellt oder bei Artefakten gegebenenfalls unterdrückt.

Werden hingegen bei 114 Einstreuungen festgestellt, erfolgt im Schritt 118 eine Analyse der Einstreuungen auf mögliche Artefakte. Im vorliegenden Fall geschieht dies über eine Analyse mittels Kapazitätsmessung.

Werden im Block 120 Artefaktsignale nicht erkannt, wird daraus geschlossen, dass es sich bei den Einstreuungen um Biosignale handelt, anderenfalls um Artefakte oder eine Kombination von Artefakten und Biosignalen.

Im Falle, dass keine Artefaktsignale vorhanden sind, wird in einem weiteren Entscheidungsblock 122 das einstreuende Signal durch Vergleich mit einem gespeicherten Muster, hier einem Epilepsie-Muster verglichen. Ist ein Vergleich des Signals mit einem gespeicherten Muster positiv, wird dieses in einem Block 124 als Epilepsie-typisches Potential angezeigt, ist dies nicht der Fall, wird einem Block 128 angezeigt, dass keine Bewertung vorgenommen wird.

Sind Artefaktsignale vorhanden, kann optional auch noch in einem Entscheidungsblock 126 geprüft werden, ob bei einer möglichen Kombination mit einem Biosignal das Biosignal noch erkannt wird und Übereinstimmungen mit einem typischen Epilepsie-Muster aufweist. Im positiven Fall kann dann ebenfalls zum Block 124 verzweigt werden und ein Epilepsie-typisches Potential angezeigt werden oder sofern dies nicht der Fall ist oder keine Erkennbarkeit von Biosignalen möglich ist, wird zum Block 128 verzweigt und keine Bewertung angezeigt.

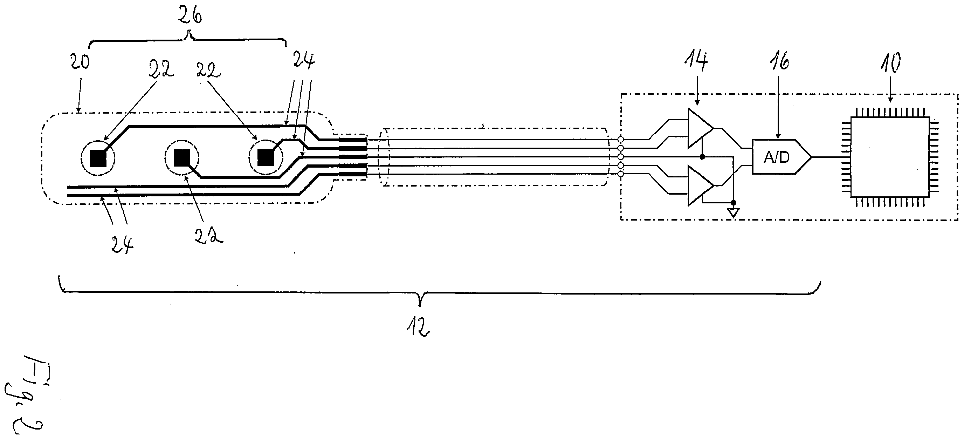

Fig. 2 zeigt eine Vorrichtung aus einem Rechner und einer Messeinheit für einen EEG-Kanal.

Die Vorrichtung besteht aus einem Rechner 10 und einer Messeinheit 12 für einen EEG-Kanal, bestehend aus einem Vorverstärker 14 mit einem Analog/Digital-Wandler 16 sowie einer Mehrelektrodenanordnung 26 auf einem Elektrodenstreifen 20 mit drei Elektroden 22 und in den Elektrodenstreifen 20 eingebetteten Leitern 24. Durch Ergänzung des Elektrodenstreifens 20 mit weiteren Elektroden 22 können Ableitungen für weitere EEG-Kanäle ergänzt werden.

Der Elektrodenstreifen 20 wird am Kopf eines Patienten angebracht und an einen Vorverstärker 14 mit Analog/DigitalWandler 16 angeschlossen. Der Vorverstärker 14 bildet die Differenzen zwischen den Potentialen der Elektroden 22 und verstärkt die Differenzpotentiale zur Wertebereichsanpassung an den Analog/Digital-Wandler 16.

Der Vorverstärker 14, der Analog/Digital-Wandler 16 und der Rechner 10 können an dem Elektrodenstreifen 20 angeordnet und mit den Leitern 24 verbunden sein und ein Anzeigesignal telemetrisch auf einen Monitor übermitteln. Alternativ kann der Rechner 10 auch ein externer Rechner sein, der telemetrisch oder drahtgebunden mit dem Vorverstärker 14 und Analog/Digital-Wandler 16 gekoppelt ist. Handelt es sich bei dem Elektrodenstreifen 20 um ein Einweg-Material, kann dieses unter Weiternutzung von Vorverstärker 14, der Analog/DigitalWandler 16 und der Rechner 10 ausgetauscht werden.

Mittels des Rechners 10 werden in bekannter Weise aus den EEG-Kurven Parameter aus dem Zeit- und/oder Frequenzbereich ermittelt, indem die ermittelten Parameter in multivariate Klassifikationsfunktionen eingesetzt werden und daraus automatisch eine Stadieneinteilung des Narkose- oder Intensiv-EEG vorgenommen wird.

Zusätzlich werden die EEG-Kurven auf Kurvenmuster durch weitere Biosignale, insbesondere Epilepsie-typische Kurvenmuster analysiert.

Mittels einer Kapazitätsmessung zwischen benachbarten Anschlussleitungen 24 der Mehrelektrodenanordnung 26 werden Artefakte als Formänderungen der Ableitungselektroden 22 erfasst. Die Kapazitätsmessung kann durch Auswertung einer vom Vorverstärker 14 verstärkten Spannungsänderung an den Leitern 24 des Elektrodenstreifen 20, die zur Kapazitätsmessung aber nicht zur EEG-Ableitung dienen, vorgenommen werden. Die Spannungsänderung rührt von einer Ladungsverschiebung her. Alternativ ist auch ein Einspeisen eines von einem Signalgenerator gelieferten Wechselspannungssignals in ein Leiterpaar der Anschlussleitungen 24 der Mehrelektrodenanordnung 26 und eine Spannungsmessung des in ein anderes Leiterpaar der Anschlussleitungen 24 der Mehrelektrodenanordnung 26 übergekoppelten Wechselspannungssignals möglich. Die jeweils gemessene Kapazitätsänderung stellt ein Maß für die Verformung der Ableitungselektroden 22 dar.

Durch Vergleich der Kapazitätsänderungen mit dem analysierten Epilepsie-typischen Kurvenmuster im EEG kann festgestellt werden, ob das Kurvenmuster durch Einkopplung von Artefakten entstanden sein könnte oder mit höherer Wahrscheinlichkeit seinen Ursprung in Gehirnstrom-Aktivität hat.

## Patentansprüche

1. Verfahren zur Auswertung eines Narkose- oder Intensiv-EEG mittels eines Rechners, der folgende Schritte durchführt:
- Ermitteln von Parametern aus dem Zeit- und/oder Frequenzbereich aus gemessenen EEG-Kurven eines mit einer Messeinheit (12) für einen EEG-Kanal mit einer Mehrelektrodenanordnung (26) erfassten EEG,
- Einsetzen der ermittelten Parameter in multivariate Klassifikationsfunktionen und Vornehmen einer automatischen Stadieneinteilung des Narkose- oder Intensiv-EEG,
- Durchführen eines ersten Analyseschrittes der gemessenen EEG-Kurven, in dem die Kurvenverläufe auf einstreuende Signalanteile untersucht werden, wobei einstreuende Signalanteile solche Signalanteile sind, die den Gesamtkurvenverlauf beeinflussen, aber untypisch für ein Narkose- oder Intensiv-EEG sind, wobei solche untypischen Signalanteile, auf die untersucht wird, aus der Menge Biosignale nicht-Narkose- oder Intensiv-EEG charakteristischer Kurven und Artefakte gebildet sind, wobei die Biosignale nicht-Narkose- oder Intensiv-EEG charakteristischer Kurven Epilepsie-typische Kurvenverläufe in Form von Krampfpotentialen beinhalten,
- Durchführen eines zweiten Analyseschrittes, der bei Nachweis solcher einstreuender Signalanteile durchgeführt wird, wobei eine Artefaktanalyse durchgeführt wird, um herauszufinden, ob es sich bei den einstreuenden Signalanteilen um Biosignale oder Artefakte handeln könnte,
- wobei bei der Artefaktanalyse anhand von von Artefaktsensoren, die als Verformungssensoren der EEG-Elektroden ausgebildet sind, erfassten Signalen die Existenz von Artefakten verifiziert wird, wenn Formänderungen mittels der Verformungssensoren erfasst werden, und die Existenz von Biosignalen nicht-Narkose- oder Intensiv-EEG charakteristischer Kurven verifiziert wird, wenn Formänderungen mittels der Verformungssensoren nicht erfasst werden.
- Darstellen des Stadiums des Narkose- oder Intensiv-EEG, wenn keine einstreuenden Signalanteile festgestellt sind,
- Nichtdarstellen des Stadiums des Narkose- oder Intensiv-EEG, wenn Artefakte festgestellt sind.

2. Verfahren nach Anspruch 1, **dadurch gekennzeichnet, dass** bei Verifizierung von Biosignalen nicht-Narkose- oder Intensiv-EEG charakteristischer Kurven die Analyse dieser Biosignale durch Vergleich der durch die einstreuenden Bio-signale generierten EEG-Kurven mit übereinstimmenden Merkmalen gespeicherter Grafikmuster von Kurvenverläufen unterschiedlichen Bioursprungs durchgeführt wird.

3. Vorrichtung zur Auswertung eines Narkose- oder Intensiv-EEG mittels eines Rechners, wobei die Vorrichtung aufweist:
- eine Messeinheit (12) für einen EEG-Kanal mit einer Mehrelektrodenanordnung (26),
- Artefaktsensoren, die als Verformungssensoren der EEG-Elektroden ausgebildet sind,
- einen Rechner, der zur Durchführung folgender Schritte eingerichtet ist:
- Ermitteln von Parametern aus dem Zeit- und/oder Frequenzbereich aus gemessenen EEG-Kurven eines mit der Messeinheit (12) erfassten EEG,
- Einsetzen der ermittelten Parameter in multivariate Klassifikationsfunktionen und Vornehmen einer automatischen Stadieneinteilung des Narkose- oder Intensiv-EEG,
- Durchführen eines ersten Analyseschrittes der gemessenen EEG-Kurven, in dem die Kurvenverläufe auf einstreuende Signalanteile untersucht werden, wobei einstreuende Signalanteile solche Signalanteile sind, die den Gesamtkurvenverlauf beeinflussen, aber untypisch für ein Narkose- oder Intensiv-EEG sind, wobei solche untypischen Signalanteile, auf die untersucht wird, aus der Menge Biosignale nicht-Narkose- oder Intensiv-EEG charakteristischer Kurven und Artefakte gebildet sind, wobei die Biosignale nicht-Narkose- oder Intensiv-EEG charakteristischer Kurven Epilepsie-typische Kurvenverläufe in Form von Krampfpotentialen beinhalten,
- Durchführen eines zweiten Analyseschrittes, der bei Nachweis solcher einstreuender Signalanteile durchgeführt wird, wobei eine Artefaktanalyse durchgeführt wird, um herauszufinden, ob es sich bei den einstreuenden Signalanteilen um Biosignale oder Artefakte handeln könnte,
- wobei bei der Artefaktanalyse anhand der von den Verformungssensoren erfassten Signale die Existenz von Artefakten verifiziert wird, wenn Formänderungen mittels der Verformungssensoren erfasst werden, und die Existenz von Biosignalen nicht-Narkose- oder Intensiv-EEG charakteristischer Kurven verifiziert wird, wenn Formänderungen mittels der Verformungssensoren nicht erfasst werden,
- Darstellen des Stadiums des Narkose- oder Intensiv-EEG, wenn keine einstreuenden Signalanteile festgestellt sind,
- Nichtdarstellen des Stadiums des Narkose- oder Intensiv-EEG, wenn Artefakte festgestellt sind.

4. Vorrichtung nach Anspruch 3, **dadurch gekennzeichnet, dass** der Rechner einen Speicher mit gespeicherten Grafikmustern von Kurvenverläufen unterschiedlichen Bioursprungs umfasst und einen programmgesteuerten Vergleich zwischen den die EEG-Kurven enthaltenden Kurvenverläufen als zweidimensionale Grafiken mit gespeicherten Grafikmustern der Kurvenverläufe unterschiedlichen Bioursprungs bei Verifizierung von Biosignalen nicht-Narkose- oder Intensiv-EEG charakteristischer Kurven vornimmt.

5. Vorrichtung nach Anspruch 4, **dadurch gekennzeichnet, dass** die Verformungssensoren Kapazitäten umfassen, die durch Verformung veränderbar sind und deren Kapazitätsänderung mit der Verformung der EEG-Elektroden korreliert.

6. Vorrichtung nach Anspruch 5, **dadurch gekennzeichnet, dass** die Kapazitäten aus benachbarten Anschlussleitungen und zwischen den Anschlussleitungen angeordneten, jeweils ein Dielektrikum darstellenden Isolierungen einer Mehrelektrodenanordnung bestehen.

7. Vorrichtung nach einem der Ansprüche 4 bis 6, **dadurch gekennzeichnet, dass** die EEG-Elektroden Hautpräparationsmittel aus Oberflächen vergrößernden Ein- und/oder Ausstülpungen der Elektrodenoberfläche umfassen.

## Claims

1. Method for evaluating an anesthesia or intensive-care EEG by means of a computer, which executes the followings steps:
- establishing parameters from the time and/or frequency domain from measured EEG curves of an EEG established by a measuring unit (12) for an EEG channel by a multi-electrode arrangement (26),
- inserting the established parameters in multi-variate classification functions and performing automatically a classification of stages of the anesthesia or intensive-care EEG,
- performing a first analysis step of the measured EEG curves, wherein the curve profiles are analyzed in respect of scattering-in signal components, wherein scattering-in signal components are those that influence the over-all curve profile but are atypical for anesthesia or intensive-care EEG, wherein such atypical signal components which are analyzed are of the bulk of bio-signals of non-anesthesia or non-intensive-care EEG characteristic curves and artifacts, wherein bio-signals of non-anesthesia or non-intensive-care EEG characteristic curves comprise epilepsy-typical curve patterns in the form of seizure potentials,
- performing a second analysis step, which is performed if such scattering-in signal components are detected, wherein an artifact analysis is performed for verifying, if the scattering-in signal components could be bio-signals or artifacts,
- wherein in the artifact analysis by means of signals which are established by artifact sensors, which are embodied as deformation sensors of the EEG electrodes, the existence of artifacts is verified, if shape modifications are established by means of the deformation sensors, and the existence of bio-signals of non-anesthesia or non-intensive-care EEG characteristic curves is verified, if shape modifications are not established by the deformation sensors,
- displaying the stage of the anesthesia or intensive-care EEG, if no scattering-in signal components are detected,
- not displaying the stage of the anesthesia or intensive-care EEG, if artifacts are detected.

2. The method of claim 1, **characterized in that** in case of a verification of bio-signals of non-anesthesia or non-intensive-care EEG characteristic curves the analysis of these bio-signals is carried out by comparing the EEG curves generated by the scattering-in bio-signals with corresponding features of stored graphical patterns of curve profiles of various bio-origins.

3. Device for evaluating an anesthesia or intensive-care EEG by means of a computer, wherein the device comprises:
- a measuring unit (12) for an EEG channel with a multi-electrode arrangement (26),
- artifact sensors which are embodied as deformation sensors of the EEG electrodes,
- a computer which is set up for executing the following steps:
- establishing parameters from the time and/or frequency domain from measured EEG curves of an EEG established by a measuring unit (12) for an EEG channel by a multi-electrode arrangement (26),
- inserting the established parameters in multi-variate classification functions and performing automatically a classification of stages of the anesthesia or intensive-care EEG,
- performing a first analysis step of the measured EEG curves, wherein the curve profiles are analyzed in respect of scattering-in signal components, wherein scattering-in signal components are those that influence the over-all curve profile but are atypical for anesthesia or intensive-care EEG, wherein such atypical signal components which are analyzed are of the bulk of bio-signals of non-anesthesia or non-intensive-care EEG characteristic curves and artifacts, wherein bio-signals of non-anesthesia or non-intensive-care EEG characteristic curves comprise epilepsy-typical curve patterns in the form of seizure potentials,
- performing a second analysis step, which is performed if such scattering-in signal components are detected, wherein an artifact analysis is performed for verifying, if the scattering-in signal components could be bio-signals or artifacts,
- wherein in the artifact analysis by means of signals which are established by artifact sensors, which are embodied as deformation sensors of the EEG electrodes, the existence of artifacts is verified, if shape modifications are established by means of the deformation sensors, and the existence of bio-signals of non-anesthesia or non-intensive-care EEG characteristic curves is verified, if shape modifications are not established by the deformation sensors,
- displaying the stage of the anesthesia or intensive-care EEG, if no scattering-in signal components are detected,
- not displaying the stage of the anesthesia or intensive-care EEG, if artifacts are detected.

4. The device of claim 3, **characterized in that** the computer comprises storage means with stored graphical patterns of curve profiles of various bio-origins and undertakes a program-controlled comparison between the curve profiles containing the EEG curves as two-dimensional graphics and stored graphical patterns of the curve profiles of various bio-origins, if the existence of bio-signals of non-anesthesia or non-intensive-care EEG characteristic curves is verified.

5. The device of as claimed in claim 4, **characterized in that** the deformation sensors comprise capacitances, which can be changed by deformation and the change in capacitance of which correlates with the deformation of the EEG electrodes.

6. The device as claimed in claim 5, **characterized in that** the capacitances consist of adjacent connection lines and insulations, respectively constituting a dielectric and arranged between the connection lines, of a multi-electrode arrangement.

7. The device as claimed in one of claims 5 to 10, **characterized in that** the EEG electrodes comprise skin preparation means made of surface-increasing invaginations and/or evaginations of the electrode surface.

## Revendications

1. Procédé d'interprétation d'un EEG sous anesthésie ou en soins intensifs au moyen d'un ordinateur qui exécute les étapes suivantes :
- détermination de paramètres appartenant au domaine du temps et/ou des fréquences à partir de courbes d'EEG mesurées d'un EEG acquis avec une unité de mesure (12) pour un canal d'EEG avec un arrangement à électrodes multiples (26),
- utilisation des, paramètres déterminés dans des fonctions de classification multivariables et réalisation d'une division en stades automatique de l'EEG sous anesthésie ou en soins intensifs,
- exécution d'une première étape d'analyse des courbes d'EEG mesurées, au cours de laquelle les tracés des courbes sont analysés pour détecter des composantes de signal dispersées, des composantes de signal dispersées étant des composantes de signal qui influencent le tracé global de la courbe, mais qui sont inhabituelles pour un EEG sous anesthésie ou en soins intensifs, lesdites composantes de signal inhabituelles qui font l'objet d'une analyse étant formées à partir de la quantité de signaux biologiques des courbes non caractéristiques d'un EEG sous anesthésie ou en soins intensifs et d'artefacts, les signaux biologiques de courbes non caractéristiques d'un EEG sous anesthésie ou en soins intensifs contenant des tracés de courbe typiques d'une épilepsie sous la forme de potentiels de spasme,
- exécution d'une deuxième étape d'analyse, avec laquelle une preuve de la présence de telles composantes de signal dispersées est apportée, une analyse d'artefacts étant exécutée afin de distinguer si les composantes de signal dispersées pourraient être des signaux biologiques ou des artefacts,
- l'existence d'artefacts étant vérifiée lors de l'analyse d'artefacts, au moyen de signaux acquis par des capteurs d'artefacts qui sont réalisés sous la forme de capteurs de déformation des électrodes d'EEG, lorsque des changements de forme sont détectés au moyen des capteurs de déformation, et l'existence de signaux biologiques de courbes non caractéristiques d'un EEG sous anesthésie ou en soins intensifs étant vérifiée lorsque des changements de forme ne sont pas détectés au moyen des capteurs de déformation,
- représentation de l'état de l'EEG sous anesthésie ou en soins intensifs lorsqu'aucune composante de signal dispersée n'est constatée,
- non-représentation de l'état de l'EEG sous anesthésie ou en soins intensifs lorsque des artefacts sont constatés.

2. Procédé selon la revendication 1, **caractérisé en ce que** lors de la vérification des signaux biologique de courbes non caractéristiques d'un EEG sous anesthésie ou en soins intensifs, l'analyse de ces signaux biologiques est effectuée en comparant les courbes d'EEG générées par les signaux biologiques dispersés avec des caractéristiques concordantes de modèles graphiques enregistrés de tracés de courbe de différentes origines biologiques.

3. Dispositif d'interprétation d'un EEG sous anesthésie ou en soins intensifs au moyen d'un ordinateur, le dispositif comprenant :
- une unité de mesure (12) pour un canal d'EEG avec un arrangement à électrodes multiples (26),
- des capteurs d'artefacts qui sont réalisés sous la forme de capteurs de déformation des électrodes d'EEG,
- un ordinateur qui est conçu pour exécuter les étapes suivantes :
- détermination de paramètres appartenant au domaine du temps et/ou des fréquences à partir de courbes d'EEG mesurées d'un EEG acquis avec l'unité de mesure (12),
- utilisation des paramètres déterminés dans des fonctions de classification multivariables et réalisation d'une division en stades automatique de l'EEG sous anesthésie ou en soins intensifs,
- exécution d'une première étape d'analyse des courbes d'EEG mesurées, au cours de laquelle les tracés des courbes d'une composante de signal sont analysés pour détecter des composantes de signal dispersées, des composantes de signal dispersées étant des composantes de signal qui influencent le tracé global de la courbe, mais qui sont inhabituelles pour un EEG sous anesthésie ou en soins intensifs, lesdites composantes de signal inhabituelles qui font l'objet d'une analyse étant formées à partir de la quantité de signaux biologiques de courbes non caractéristiques d'un EEG sous anesthésie ou en soins intensifs et d'artefacts, les signaux biologiques de courbes non caractéristiques d'un EEG sous anesthésie ou en soins intensifs contenant des tracés de courbe typiques d'une épilepsie sous la forme de potentiels de spasme,
- exécution d'une deuxième étape d'analyse, avec laquelle une preuve de la présence de telles composantes de signal dispersées est apportée, une analyse d'artefacts étant exécutée afin de distinguer si les composantes de signal dispersées pourraient être des signaux biologiques ou des artefacts,
- l'existence d'artefacts étant vérifiée lors de l'analyse d'artefacts, au moyen des signaux acquis par les capteurs de déformation, lorsque des changements de forme sont détectés au moyen des capteurs de déformation, et l'existence de signaux biologiques de courbes non caractéristiques d'un EEG sous anesthésie ou en soins intensifs étant vérifiée lorsque des changements de forme ne sont pas détectés au moyen des capteurs de déformation,
- représentation de l'état de l'EEG sous anesthésie ou en soins intensifs lorsqu'aucune composante de signal dispersée n'est constatée,
- non-représentation de l'état de l'EEG sous anesthésie ou en soins intensifs lorsque des artefacts sont constatés.

4. Dispositif selon la revendication 3, **caractérisé en ce que** l'ordinateur comprend une mémoire dans laquelle sont enregistrés des modèles graphiques de tracés de courbe de différentes origines biologiques et effectue une comparaison commandée par programme entre les tracés de courbe contenant les courbes d'EEG sous la forme de graphiques en deux dimensions et les modèles graphiques enregistrés de tracés de courbe de différentes origines biologiques lors de la vérification de l'existence de signaux biologiques de courbes non caractéristiques d'un EEG sous anesthésie ou en soins intensifs.

5. Dispositif selon la revendication 4, **caractérisé en ce que** les capteurs de déformation comprennent des capacités qui sont modifiables par la déformation et dont la variation de capacité est corrélée avec la déformation des électrodes d'EEG.

6. Dispositif selon la revendication 5, **caractérisé en ce que** les capacités se composent de lignes de raccordement voisines et d'isolations disposées entre les lignes de raccordement, représentant respectivement un diélectrique, d'un arrangement à électrodes multiples.

7. Dispositif selon l'une des revendications 4 à 6, **caractérisé en ce que** les électrodes d'EEG comprennent des moyens de préparation cutanée composés de surfaces de cavités et/ou de protubérances agrandies de la surface des électrodes.
